# EUROPEAN PATENT APPLICATION

(11) **EP 2 064 995 A1**
(43) Date of publication of application: **03.06.2009**
(21) Application number: 06798371.8
(22) Date of filing: 27.09.2006
(51) Int. Cl.: A61B 8/08

(54) **ULTRASONIC BREAST DIAGNOSTIC SYSTEM**

(30) Priority: 22.09.2006 JP 2006257169
(71) Applicant: Aloka Co., Ltd., Mitaka-shi Tokyo 181-8622 (JP)
(72) Inventor: FUJITA, Hiroshi, Gifu-shi Gifu 501-1193 (JP); FUKUOKA, Daisuke, Gifu-shi Gifu 501-1193 (JP); HARA, Takeshi, Gifu-shi Gifu 501-1193 (JP); KATO, Keiji, Mitaka-shi Tokyo 181-8622 (JP)
(74) Representative: Evans, Huw David Duncan
(86) International application number: PCT/JP2006/319164
(87) International publication number: WO 2008/035444

(57) **Abstract**

[PROBLEMS] To provide an ultrasonic breast diagnostic system suitable for screening of breast cancer and minimizing the burden on the doctor examining the captured image. [MEANS FOR SOLVING PROBLEMS] An ultrasonic breast diagnostic system comprises an ultrasonic breast imaging apparatus (10) having a water bath (11) into which left and right breasts can be dipped downward and an ultrasonic probe (12) so disposed on the bottom of the water bath (11) to mechanically scan and adapted to three-dimensionally image the whole region of the breasts by transmitting/receiving an ultrasonic wave, a breast voxel data creating device (20) for creating voxel data on the entire breasts from image data (A) acquired by the apparatus (10), and an examination image display (30) for displaying an image for image examination from the voxel data on the breasts created by the device (20); which sequentially displays tomograms of cross-sections at bilaterally symmetrical positions of the breasts side by side symmetrically at predetermined pitches from one end of the breast to the other.

## Description

### Technical Field

The present invention relates to an ultrasound breast diagnostic system for diagnosing the presence of a lesion portion in a breast region by using ultrasound waves and particularly to an ultrasound breast diagnostic system suitable for use in screening of breast cancer.

### Background Art

Breast cancer ranks first in cancer morbidity (female) and its early detection is one of major objects in the medical field. Recently, examination by mammography, which is an X-ray transmission image, has been used as effective means for early detection. Using this X-ray mammography, not only small tumors that can not be detected by palpation but also fine calcification which might be malignant can be detected.

On the other hand, radiogram examination diagnosis by using an ultrasound image (tomographic image) obtained by transmission / reception of ultrasound waves, that is, ultrasound examination is non-invasive means and has been also known as effective particularly for diagnosis of a tumor. Furthermore, if the ultrasound examination is to be applied to screening of breast cancer, it is important to pick up a full image of an entire breast region and various proposals relating to technologies with such a purpose have been made.

A typical example is a mammary ultrasound image pickup method called a "water bath type" described in Patent Document 1, for example, in which a breast is immersed downward in a water bath, and the entire breast region is imaged by performing mechanical scanning with an ultrasound probe arranged below it. The water bath type is characterized in that though the breast is immersed in the water with a soft thin film interposed therebetween in order to retain a shape of the breast, the entire breast can be imaged in a substantially natural bulge. According to the document, an obtained ultrasound image (B-mode image) is displayed on a monitor as a moving image or recorded once and then, used for image diagnosis by being replayed and displayed.

Also, according to the "water bath type", since an obtained C-mode image is a coronal image, which is a cross-section of a breast, Patent Document 2 discloses a method for obtaining an appropriate number of C-mode images (coronal images) with a predetermined pitch from a mammilla to a breast bottom portion and use of them for screening of breast cancer.

Also, unlike the above technology in which a probe is used for mechanically scanning the breast in a non-contact manner using water as an acoustic coupling medium, an ultrasound image pickup method of mechanically scanning with a probe in contact with the surface of the breast is well known. Patent Document 3 is a typical example, which discloses a technology in which an ultrasound probe provided with a position sensor is mechanically scanned along the surface of a breast of a subject positioned face up or on their stomach so as to image a breast region. The mechanical scanning of the probe is for scanning at a uniform speed along the surface of the breast, but the mechanical scanning is performed in 5 rows specifically for the scanning of the entire region of the breast. In addition, a large number of ultrasound images (B-mode images) picked up with a predetermined pitch for each row are recorded once and then, continuously displayed for image diagnosis. With this type of image pickup method, since the scanning of the probe is performed under a certain pressure so as to maintain a close contact state with the breast surface, an obtained image is different from an original shape of the breast.

Patent Document 4 discloses a technology art, though having been already put into practice, each image (transmission image) of right and left breasts are placed "with back to back" in image diagnosis by the X-ray mammography, that is, the breasts are displayed adjacently and symmetrically with the breast base portion sides abutting each other. The document relates to positioning of the right and left breast images in the adjacent display.
Patent Document 1: Japanese Unexamined Patent Application Publication No. 2002-336256 (Fig. 1, paragraph [0021])
Patent Document 2: Japanese Unexamined Patent Application Publication No. 58-58033 (Figs. 1, 4, page 3, upper right column, lines 8 to 14)
Patent Document 3: Japanese Unexamined Patent Application Publication (Translation of PCT Application) No. 2004-516865 (Figs. 1 to 5, paragraph [0052])
Patent Document 4: Japanese Unexamined Patent Application Publication No. 2006-061472 (Fig. 3, paragraph [0004])

### Problems to be Solved by the Invention

Various proposals on the technology in which image diagnosis with ultrasound waves is applied to screening particularly of breast cancer have been made as mentioned above. However, despite these proposals, the ultrasound examination is not used as primary screening means of breast cancer but remains at a level of real-time examination by a hand-probe scanning, re-confirming means of a lesion portion or a positive candidate portion found by palpation or X-ray mammography or means for determining necessity of biopsy at present.

One of the reasons is that interpretation of an ultrasound image itself is not easy. That is, since inside a breast is made up of fine and dense tissues in which mammary glands, fat tissue, connective tissues and moreover, blood vessels and nerves are combined together, an ultrasound image based on a sound-ray echo generated between tissues with different acoustic impedances is formed as an image with a variety of textures, and moreover, the image is different depending on the person and the age. Also, the inside the breast is a so-called acoustic chamber, and shades such as speckle noise based on an interference wave and artifact by multiple reflection, not representing a tissue structure of a breast, are generated. Such unique ultrasound images can not be examined easily without sufficient skills.

Also, with the ultrasound image, unlike the X-ray mammography representing the entire breast in a single image, the number of images requiring interpretation is large, which is also a major reason. That is, the ultrasound image representing a fine and dense tissue structure of the breast can not be three-dimensionally image-processed like MIP (MinIP) processing, and a large number of tomograms should be interpreted in order to examine the entire breast for screening. For example, when a breast region with an entire width of 16 cm is to be examined by a 2mm-pitch cross section, 80 ultrasound images (tomograms) need to be interpreted, and if the probe scanning is performed with 5 rows as in the above Patent Document 3, as many as 400 images in total need to be interpreted. This is an extreme burden for doctors in giving diagnosis that a lesion portion as an abnormal portion is discovered or no lesion portion is discovered.

The ultrasound examination has not been applied to the screening of breast cancer at least in a full scale as above mainly because non-easiness of the examination and the large number of images specific to the ultrasound image. However, the examination by X-ray mammography has a problem not only of radiation exposure but also of inability of accurate image diagnosis of dense breasts prevalent in Japanese women. Also, the X-ray photography (compression method) made in a state where a breast region is pulled out to the body surface side and sandwiched and tightened between upper and lower or right and left plates is more or less painful for women not only in a young generation, which is another problem. Thus, the breast cancer screening by ultrasound waves still attract high expectations particularly in our country where examination on the young generation is also being discussed (The breast cancer screening by MRI (magnetic resonance imaging) has been known recently, but MRI needs contrasting, and examination is not easy. Thus, MRI is suitable for examination on progress of a lesion (higher examination) but not for screening (primary examination, screening)).

An object of the present invention is to provide an ultrasound breast diagnostic system particularly suitable for breast cancer screening and an ultrasound breast diagnostic system that can minimize a burden on a doctor interpreting an image by displaying an ultrasound image in an optimal form for interpretation of image.

### Means for Solving the Problems

The present invention was made by focusing on the fact that comparison with an image of a normal tissue as a reference image is the most effective in detecting abnormality or diagnosis as no abnormality in a mammary tissue but in an ultrasound image with a variety of textures and a large difference depending on the portion or the person, an image at a cross-sectional spot bilaterally symmetric to an image of a target to be diagnosed is the most suitable for the reference image.

That is, an ultrasound breast diagnostic system of the present invention in order to solve the above problems comprises the following devices, which are independent from each other or integrated as appropriate:
(a) an ultrasound breast image pickup device provided with a water bath in which a breast can be immersed downward and an ultrasound probe arranged at a bottom portion of the water bath, capable of performing mechanical scanning, for imaging an entire region of each of right and left breasts in a three-dimensional manner by transmission / reception of ultrasound waves.
(b) a breast voxel data creating device for creating voxel data of each of the entire right and left breasts on the basis of image data obtained by the ultrasound breast image pickup device.
(c) an display device used for interpretation of images for displaying an image for image diagnosis on the basis of voxel data of each of the right and left breasts created by the breast voxel data creating device in which tomograms of bilaterally symmetric cross-sectional spots of the right and left breasts are sequentially displayed adjacently symmetrically and with a predetermined pitch from one end side to the other end side (claim 1).

Here, the ultrasound breast image pickup device in the above (a) is a known "water bath type" image pickup device in the above Patent Documents 1 and 2 and the like. With this type of devices, a breast is immersed in water with a thin film such as a rubber film interposed in order to retain its shape in general, but the shape retention is not necessarily required. According to this device, since the probe scanning is performed not in contact with the breast, three-dimensional image data of the entire breast in a round form close to natural can be obtained. As a probe depth of transmission / reception of ultrasound waves by a probe, a sufficient depth that reaches pectoral muscle and rib is appropriate, and the depth can be generally set to approximately 10 cm including an interposed water phase.

Considering personal difference in screening, the mechanical scanning range of the probe in this device may be defined as a section of approximately 16 cm, for example. Thus, the probe provided in this device may have a length of approximately 16 cm so as to realize the mechanical scanning in 1 pass. However, more practically, use of a probe with a scan width of 5 to 6 cm similar to a hand probe of a general-purpose ultrasound diagnostic device is preferable, which is also preferable in a point that no special change in control circuit is needed and a freedom in probe scanning can be obtained. In this case, the mechanical scanning of the probe is performed in plural passes with an appropriate overlap.

The breast voxel data creating device in the above (b) is constituted by a computer device provided with a memory for temporarily storing image data obtained by the device (a) and produces single three-dimensional image data of each of the entire right and left breasts on the basis of the image data and is particularly important when the mechanical scanning of the probe by the ultrasound breast image pickup device (a) is performed in plural passes. More specifically, slice image data of each row obtained with a predetermined pitch along with position data of the probe by the device (a) are synthesized with each other while the overlap portions are overlapped by the breast voxel data creating device (b) and finally created preferably as isotropic voxel data. The synthesis of the overlap portions can be carried out by simple selection and deletion of one of data on the basis of coordinate data but such a method is preferable that the portions are added with weighting of inclination from one side to the other side and divided into equal two parts so that a border line is not visualized.

The preferably isotropic voxel data of the entire breast thus formed includes a peripheral region of the breast since the breast is imaged slightly widely by the ultrasound breast image pickup device (a). Thus, in the breast voxel data creating device (b), processing to delete the breast peripheral region and to create voxel data of the genuine breast region can be executed. However, the image of the breast peripheral region does not interfere with the image diagnosis but to the contrary, can serve as a reference, and such processing is not necessary in general. The voxel data of each of the right and left breasts is used for displaying an image at a cross section in an arbitrary direction for image diagnosis and is also used for three-dimensional automatic detection of a lesion portion in computer-aided diagnosis (CAD), which will be described later.

The display device used for interpretation of images in the above (c) is constituted by a computer-controlled display device and displays cross-sectional images of bilaterally symmetrical spots of the right and left breasts adjacently and bilaterally symmetrically on the basis of the voxel data of each of the right and left breasts created by the breast voxel data creating device (b) and also sequentially displays the images with a predetermined pitch from one end side to the other end side of the breast.

Here, an image displayed for image diagnosis (tomogram) may be an image of a cross section in an arbitrary direction (eye direction). However, for the voxel data having coordinate axes in the vertical direction, bilateral direction, and anteroposterior direction of a body, a longitudinal sectional plane (section dividing the breast into right and left on a front view, that is, sagittal), a transverse plane (section dividing the breast vertically, that is, axial) or a planar section (section dividing the breast in the anteroposterior direction in sliced rounds on a front view, that is, coronal) not requiring interpolation is preferable. The most preferable among them is the sagittal image and the axial image, and in the case of these tomograms, the right and left breast tomograms are adjacently displayed bilaterally or vertically symmetrically with the base portion sides of the breasts abutting each other ("back to back") (claim 2). According to this, the image of the base portion of the breast is visualized as a dark portion substantially without echo by the rib, but the right and left breast tomograms are displayed substantially as an integral breast image, and both images can be compared with each other with a minimum eye movement.

However, though the coronal image is inferior to the sagittal or axial image in a point that a relative large eye movement is required in comparative diagnosis, the coronal image has an advantage that the number of images requiring interpretation can be small since it is a genuine tomogram of the breast region. Also, the coronal image of the breast is not suitable for determination of a state of rearward echo of a tumor but it is extremely suitable for diagnosis of disturbance in the structure of a certain tissue in an initial state of cancer. Therefore, the tomograms of the right and left breasts adjacently displayed bilaterally symmetrically in the display device used for interpretation of images (c) is also preferably the coronal image (claim 3).

In the device (c), such tomograms are sequentially displayed with a predetermined pitch from one end side to the other side of a breast region in order to examine the entire region of the breast exhaustively on the presence of a lesion and the like. Here, the pitch of the section can be determined as appropriate according to a size of a lesion portion to be detected, and if a tumor of approximately 5 mm or more is required to be detected, a pitch of approximately 2 to 4 mm is appropriate. Though depending on the voxel size, the smaller this pitch is set, the more precise interpretation of images becomes possible, but the number of images to be interpreted is increased. A speed of sequential display of the tomograms is preferably possible to be set as appropriate by an examiner. Also, in the continuous display, display indicating a section in what spot in the breast the tomogram in display refers to is also preferably made on a screen. The display of the cross-sectional spot may be constituted as a line or a bar crossing a breast mark represented as a circle, for example, in a sectional direction.

It is important that the tomograms of the right and left breasts sequentially displayed symmetrically adjacently as above are bilaterally symmetric images, that is, images of the same cross-sectional spot on the premise that the breasts are not much different in size and form but bilaterally symmetric. As a result, the right and left breast tomograms can be the best reference image in examining a lesion or abnormality in a tissue.

According to the water bath type ultrasound breast image pickup device in the above (a), since each of the right and left breasts is imaged in an attitude that the mammilla is located at the center of the water bath in general, the bilaterally symmetric spots of the right and left breasts can be acquired from coordinate values as they are. However, actually, the positions of the breasts in imaging are somewhat different, and even if the three-dimensional shape of the breast itself is the same between the right and left breast images, there is some displacement and the like. Then, positions of their mammillas are detected on the basis of the voxel data of the right and left breasts, respectively, and the bilaterally symmetric spots of the right and left breasts are preferably bilaterally symmetric spots using the mammilla as a reference point, or more specifically, preferably the bilaterally symmetric spots separated by the same distance from the mammilla of each of the right and left breasts or rather indispensable in the case of the coronal image (claim 4). By using the mammilla as a reference, there will be no corresponding bilaterally symmetric spots at an end of the breast region, but in this case, such a display method can be taken that one of the images is stopped till the bilaterally symmetric spots match each other, for example.

Apart from the symmetric display of the tomograms of the right and left breasts as above, in the display device used for interpretation of images (c), the three-dimensional display and the like of any abnormal portion, if detected, can be made. More details and modes relating to such display used for interpretation of images will be described later as the best mode for carrying out the invention.

Moreover, a system known as CAD can be introduced to the ultrasound breast diagnostic system of the present invention. That is, the ultrasound breast diagnostic system of the present invention is further provided with the following device:
(d) a lesion portion automatic detecting / marking device that analyzes the voxel data of each of the right and left breasts by computer, automatically detects a lesion portion as a positive candidate and displays a mark indicating the positive candidate overlaid on the image (claim 5).

Here, the lesion portion is specifically a tumor, and a method for automatically detecting the tumor includes a binarization method, a method on the basis of concentration degree of concentration gradient vector, a method using a three-dimensional Gaussian-Laplace (LoG) filter, a method using texture analysis and the like. In a preferred embodiment, which will be described later, initial detection of a tumor is performed by the binarization method, with which the processing can be executed most easily. Alternatively, the mark indicating the positive candidate of the detected lesion portion may be an arrow whose tip end is directed to the positive candidate or a surrounding line surrounding the positive candidate as having been well-known.

According to the lesion portion automatic detecting / marking device (d), since the lesion portion is displayed as the positive candidate on the interpretation image, a doctor can interpret the images referring to that. This further helps alleviate a burden on doctors particularly in screening requiring careful attention so as not to miss a lesion portion.

### Advantages of the Invention

According to the ultrasound breast diagnostic system of the present invention, tomograms at the bilaterally symmetric cross-sectional spots of the right and left breasts are displayed for image diagnosis. Thus, a doctor can diagnose the presence of a lesion and the like not only from shades on the image but also through comparison with the most similar tomogram in terms of tissue, which is an image of the bilaterally symmetric image. Also, since the comparison of the tomograms is displayed adjacently in bilateral symmetry, the comparison can be made the most effectively with the minimum eye movement. That is, the comparison with the best reference image can be made under the optimal image interpretation environment. On the other hand, since the displayed tomogram is a tomogram of the entire breast, the number of tomograms required for diagnosis of the entire breast region can be minimized. Moreover, since the tomograms of the right and left breasts are displayed together, the right and left breasts can be diagnosed at a time.

Therefore, according to the ultrasound breast diagnostic system of the present invention, since the tomograms of the bilaterally symmetric cross-sectional spots of the right and left breasts are displayed symmetrically adjacently so that comparison diagnosis can be made using each other as the best reference image, accurate image diagnosis can be made with less burden even with the ultrasound image whose examination itself is not easy. Thus, the ultrasound breast diagnostic system of the present invention can minimize the burden on doctors in the interpretation of images and can be effectively applied to the screening of breast cancer.

### Best Mode for Carrying Out the Invention

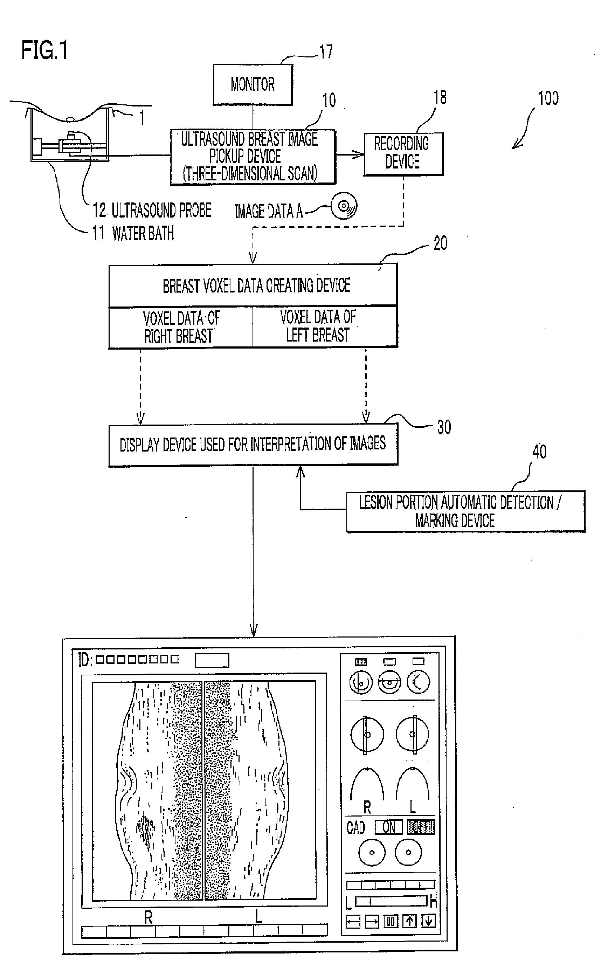

A preferred embodiment of an ultrasound breast diagnostic system of the present invention will be described below referring to the attached drawings. Fig. 1 is an explanatory diagram illustrating an outline configuration of the entire ultrasound breast diagnostic system of an embodiment of the present invention.

An ultrasound breast diagnostic system 100 of this embodiment is adapted for breast cancer examination using ultrasound waves and is provided with, as shown in Fig. 1, a water bath 11 in which a breast can be immersed downward, an ultrasound probe 12 arranged on a bottom portion of the water bath 11, capable of performing mechanical scanning in a horizontal plane, an ultrasound breast image pickup device 10 that images an entire region of each of the right and left breasts in a three-dimensional manner by transmission / reception of ultrasound waves, a breast voxel data creating device 20 that creates voxel data of each of the entire right and left breasts on the basis of image data A obtained by the ultrasound breast image pickup device 10, and an display device used for interpretation of images 30 for displaying an image for image diagnosis on the basis of voxel data of each of the right and left breasts created by the breast voxel data creating device 20, that sequentially displays tomograms at bilaterally symmetric cross-sectional spots of the right and left breasts symmetrically adjacently with a predetermined pitch from one end side to the other end side of the breast.

Moreover, the ultrasound breast diagnostic system 100 is further provided with a lesion portion automatic detecting / marking device 40 that analyzes the breast voxel data if each of the right and left breasts with a computer, automatically detects a lesion portion as a positive candidate and displays a mark indicating the positive candidate overlaid on an applicable image for image diagnosis by the display device used for interpretation of images 30.

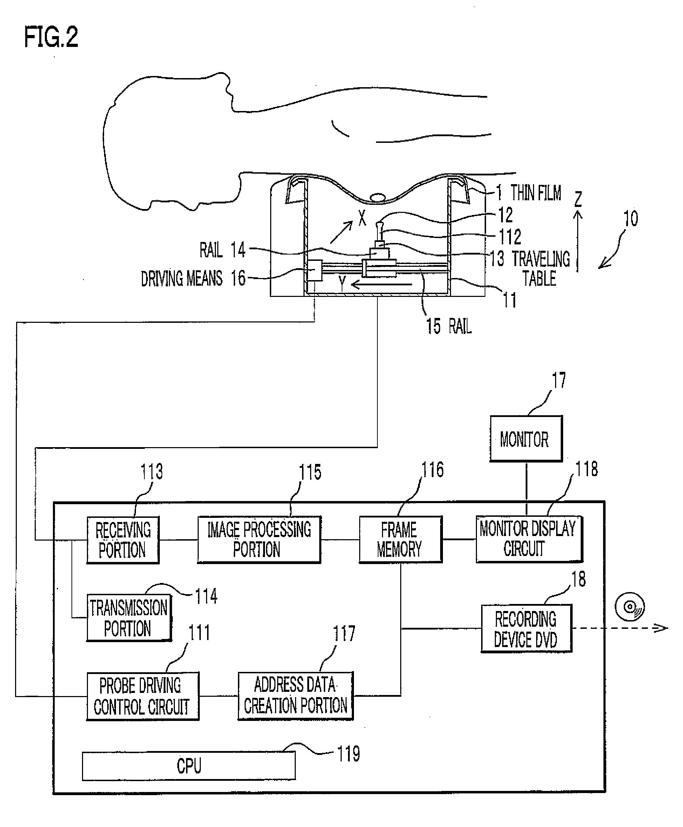

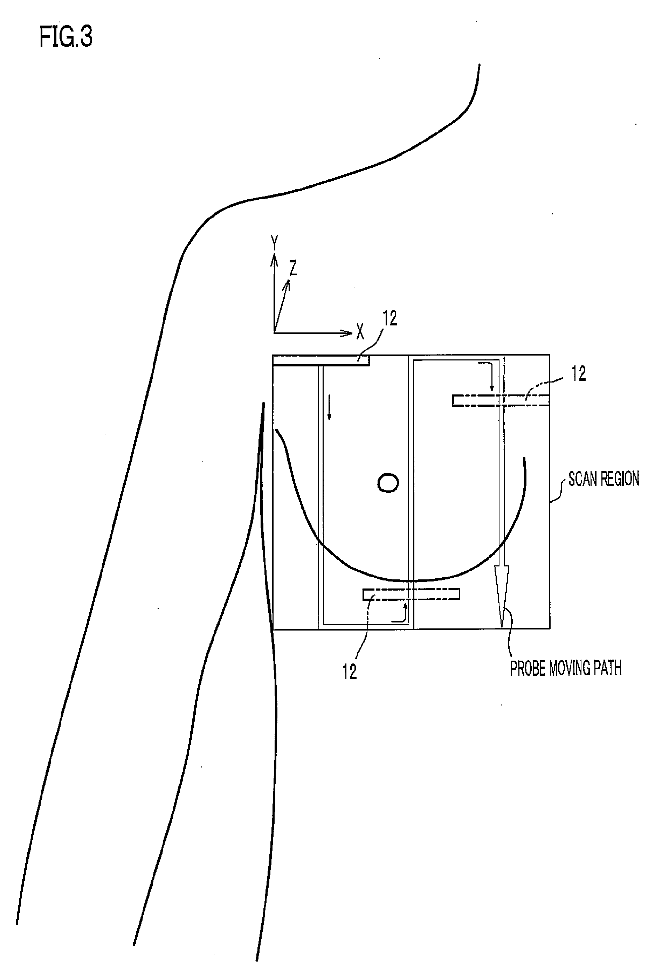

Fig. 2 is an explanatory diagram illustrating an outline configuration of the ultrasound breast image pickup device 10. Fig. 3 is an explanatory diagram illustrating a mechanical scanning path of the ultrasound probe. The ultrasound breast image pickup device 10 is a so-called "water bath type" image pickup device and as shown in Fig. 2, picks up an ultrasound image of the entire breast region of a subject in a three-dimensional manner when the subject hunches over an upper opening portion of the water bath 11 and the ultrasound probe 12 arranged on the bottom portion of the water bath 11 is mechanically scanned. In Figs. 2 and 3, the X direction is the same direction as an electronic scanning direction of the ultrasound probe 12 and also a bilateral direction of the subject in this figure. The Y direction is a cephalocaudal direction of the subject, and the Z direction is a probe depth direction of transmission / reception of the ultrasound waves by the ultrasound probe 12. For accurate incidence of the ultrasound waves into the breast, the mechanical scanning of the ultrasound probe 12 may be carried out on a curved rail corresponding to the shape of the breast.

The ultrasound breast image pickup device 10 is provided with the water bath 11 having the upper opening capable of receiving either one of the right and left breasts of the subject and holding water for ultrasound propagation between the ultrasound probe 12 provided inside and the breast, a traveling base 13 on which the ultrasound probe 12 is fixed, a first rail 14 for movably guiding the traveling base 13 in the X direction, which is the same as the electronic scanning direction of the ultrasound probe 12, a second rail 15 for guiding the first rail 14 in the Y direction orthogonal to the X direction, and driving means 16 for driving the traveling base 13 along the first rail 14 and the second rail 15. The driving means 16 is made up of a stepping motor. In this embodiment, a thin film 1 with elasticity extends over the upper opening of the water bath 11 so as to cover the opening for retaining the shape of the breast.

The device 10 functionally includes a probe driving control circuit 111 for controlling the entire device, an ultrasound probe 112 for transmission / reception of ultrasound waves to / from the subject, a receiving portion 113 and a transmission portion 114 as an ultrasound transmitting / receiving device for obtaining sound-ray data of a tomogram of the subject through transmission / reception of ultrasound waves from the ultrasound probe 112, an image processing portion 115 for processing the obtained ultrasound image, a frame memory 116 in which the sound-ray data of the tomogram obtained by the image processing portion 115 is sequentially stored, an address data creation portion 117 for receiving an instruction from the probe driving control circuit 111 and supplying address data to the frame memory 116, a monitor display circuit 118 for displaying the tomograms sequentially stored in the frame memory 116 on the monitor 17 for check, a CPU 119 for entire control, and a recording device 18 for reading out an image frame from the frame memory 116 and recording it with the address data.

Here, the ultrasound probe 12 can be mechanically moved in the X direction and the Y direction at a lower part in the water bath 11. That is, automatic scan is performed mechanically in a plane substantially parallel with the body axis away from the breast by a predetermined distance. In this scanning, since the driving means 16 is made up of a stepping motor, the CPU 119 can obtain the address data in driving control of the ultrasound probe 12 in the scan. Also, since the size of the breast of a subject is different depending on the person, the scan region of the ultrasound probe 12 is made to be in a range of 16 cm vertically and laterally. Tomographic data up to 10 cm including the interposed water phase can be obtained in the depth direction. A slice pitch of the tomographic data can be set as appropriate, but considering that the voxel data to be created in a subsequent process is preferably composed of isotropic voxels, a slice pitch set in accordance with the voxels is preferable.

The scan width (array length of the transducers) of the ultrasound probe 12 is 6 cm. Thus, as shown in Fig. 3, the scanning of the ultrasound probe 12 is made in three passes including an overlap portion of approximately 1 cm. The tomograms of cross-sectional images recorded in an appropriate recording medium by the recording device 18 are stored for a predetermined period as medical images. They are also used for separately creating tomograms for interpretation of images to be performed later by using the breast voxel data creating device 20 in this embodiment.

According to the ultrasound breast image pickup device 10, in a state where the subject hunches over the upper opening portion of the water bath 11, the ultrasound probe 12 arranged on the bottom portion of the water bath 11 is mechanically scanned under control of the CPU 119 and picks up a plurality of tomograms in a predetermined region (16 cm in width and length x 10 cm in depth) of sufficient size to include the entire breast region of the subject, and the breast can be three-dimensionally imaged substantially in a natural shape.

The breast voxel data creating device 20 creates voxel data on the basis of the tomographic data A by probe scanning performed in three passes. The breast voxel data creating device 20 is made up of a computer device and creates slice image data of each row obtained at a predetermined pitch by overlapping and synthesizing the overlap portions with each other and finally making them into voxel data along with position data of the ultrasound probe 12 by using the ultrasound breast image pickup device 10 by executing an installed program.

The created voxel data is preferably composed of isotropic voxels. A method of synthesizing the overlap portions is preferably a weighted average method in which "weighting" inclined from one side to the other of an overlap portion is given, not a simple average of pixel values of the applicable spot. The voxel data created as above is held in a storage medium such as a memory of the breast voxel data creating device 20 and used as voxel data of each of the right and left breasts as appropriate in processing of the display device used for interpretation of images 30. The voxel data created by the device 20 may be recorded in a recording medium such as a HDD, DVD or the like. Fig. 1 explains an on-line media method.

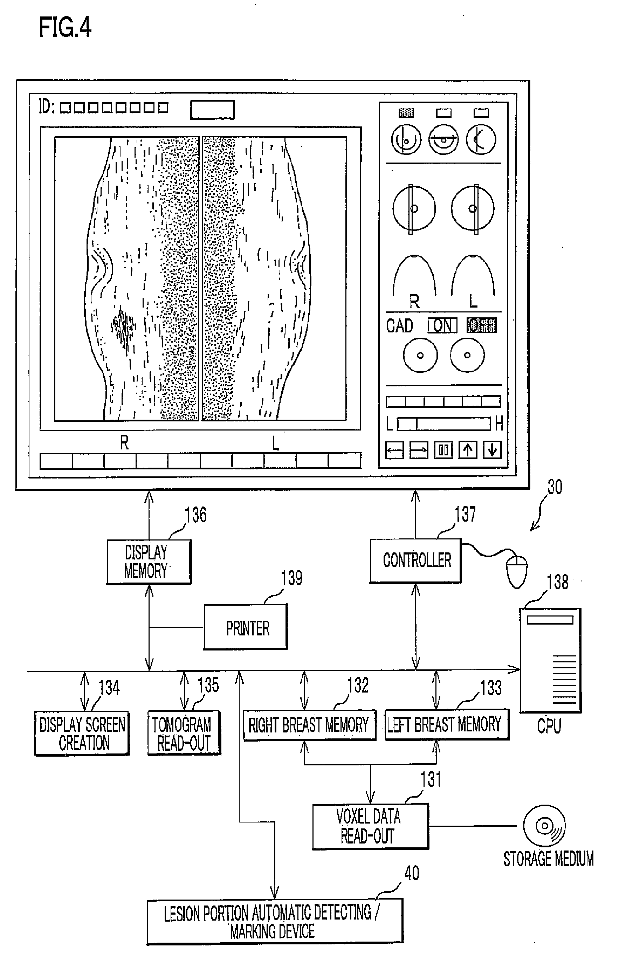

The display device used for interpretation of images 30 displays an image for image diagnosis on the basis of voxel data of each of the right and left breasts created by the breast voxel data creating device 20, and the device sequentially displays tomograms at bilaterally symmetric cross-sectional spots in the right and left breasts symmetrically adjacently and from one end side to the other end side with a predetermined pitch. Its hardware configuration is provided with, as shown in Fig. 4, voxel data read-out 131 for reading out the voxel data from the storage medium, a right-breast memory 132 and a left-breast memory 133 into which the voxel data of the right and left breasts read out by the voxel data read-out 131 is written, display image creation 134 and tomogram read-out 135 for displaying the tomogram in the predetermined direction on the basis of the right and left voxel data, a display memory 136 in which a display screen for interpretation of images created thereby is written, a controller 137 for interface between an image examiner and the device 30, and a CPU 138 for controlling them through a data bus. The lesion portion automatic detecting / marking device 40 and the display device used for interpretation of images 30 are connected to each other through the data bus. The device 30 is also provided with a printer 139 for printing a display image of the display memory 136 so that the display image to be interpreted can be printed.

Display processing of a primary image by the display device used for interpretation of images 30 is carried out by the CPU 138executing the installed program as follows. That is, the voxel data of the entire right and left breasts created by the breast voxel data creating device 20 is read out by the voxel data read-out 131 and written into the right breast memory 132, the left breast memory 133, respectively. The data in the memories 132, 133 is read out by the tomogram read-out 135 as appropriate, a tomogram in the right-side predetermined direction and a tomogram of the left-side cross-sectional spot corresponding to that tomogram are selected, the tomograms are incorporated into the display screen created by the display screen creation 134 and the screen for examination is constituted. Furthermore, the examination screen is written into the display memory 136 and displayed as shown in Fig. 4.

The screen for examination shown in Fig. 4 is divided into a plurality of windows (rectangular regions), which include an "R" region displaying the right-side breast tomogram, an "L" region displaying the left-side breast tomogram of the subject, and a region including a list displaying a sectional position of the breast and an operation panel for operation by an examiner such as ON, OFF and the like of CAD (lesion portion automatic detecting / marking device), for example. The tomograms for examination incorporated into the regions are the tomograms of each of the right and left breasts of the same subject, and the left breast tomogram is displayed in the "L" region on the display screen for examination substantially at the center of the screen, while the tomogram of the right breast corresponding to the tomogram is displayed in the "R" region substantially in the left thereof.

Here, in processing to select the bilaterally symmetric cross-sectional spots of the right and left breasts, mammilla detection processing can be carried out. The mammilla is imaged in a state buried in the breast in general under the shape retention by the thin film 1, and because of that, the mammilla is not necessarily located at the uppermost point on the breast surface. Thus, the mammilla detection processing on the basis of the breast voxel data can be carried out easily by the following method using the fact that a shade is generated in the periphery of the mammilla buried in the breast in the three-dimensional breast image. That is, a search box of such a size that it contains the mammilla region is set at the top portion (center portion) of the three-dimensional breast image, a concentration average value of all the voxels (excluding the water phase) in the search box at each point is acquired while the search box is sequentially moved, and the center of the search box at a portion where the concentration average value is the minimum is determined as the mammilla center. This method is easy in terms of processing among other things and sufficient correctness can be obtained.

In the image diagnosis displayed adjacently and bilaterally symmetrically through the above processing, the sagittal image (See Fig. 4) or axial image is provided as a primary image, and the tomograms of the right and left breast are displayed adjacently "back to back", that is, the right and left tomograms are displayed having the rib sides (breast base portion sides) made of a dark portion abutting each other bilaterally symmetrically (See Fig. 4) or vertically symmetrically. According to this, the tomograms of the right and left breasts are displayed as if an integral breast image, and both images can be compared with each other with the minimum eye movement.

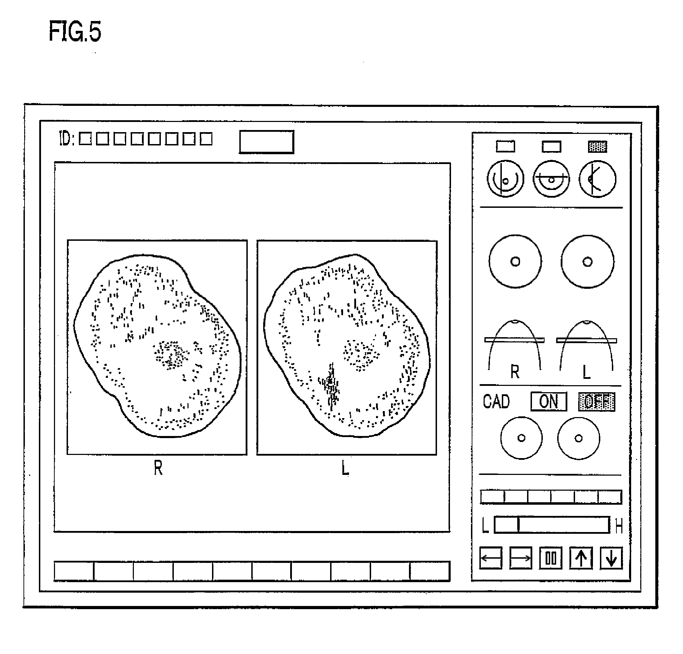

However, a coronal image is also a recommended tomogram and is extremely suitable for diagnosis of disturbance in the structure of a tissue, which is an initial state of cancer. Fig. 5 shows an example of another display screen displayed by the display device used for interpretation of images 30. In the display example shown in this figure, the coronal images are displayed bilaterally symmetrically in the right and left in parallel for image diagnosis. According to this, since the genuine breast region is displayed bilaterally symmetrically, it has an advantage that the number of images required for examination can be small.

In a window of the display device used for interpretation of images 30 displaying the breast, tomograms of the breast from axial and other arbitrary directions, not shown, can be displayed. For example, the window is divided into an upper stage in the screen and a lower stage in the screen, and the current right and left breasts of the subject may be displayed on the upper stage in the screen, while the past right and left breasts of the same subjects may be displayed on the lower stage in the screen in parallel, and each of the right and left breasts may be displayed symmetrically adjacently. In this case, there may be a window for displaying a differential image between the current and past breasts. These various screen display contents can be selected as appropriate by the examiner such as a doctor and the like by clicking on the operation panel in the window or the screen may be made single so that the section is switched as necessary.

For the right and left images displayed by the display device used for interpretation of images 30, since the tomograms of bilaterally symmetric cross-sectional spots of the right and left breasts are displayed symmetrically adjacently, comparison can be made with the best reference image when the image examiner examines a lesion or a change in a tissue on an image. There might be an unignorable difference between the right and left images in the entire shape such as a flattening degree due to a change in the shape retaining condition and the like. And if the entire shape is different, it is difficult to specify the corresponding cross-sectional spot. In such a case, the display device used for interpretation of images 30 employs a display method in which one of the images is stopped till the right and left symmetric spots match each other.

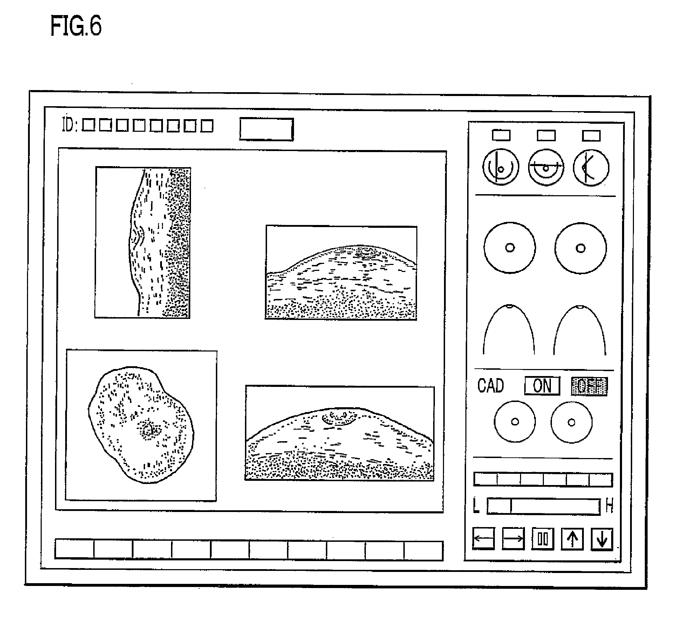

Fig. 6 is a diagram illustrating an example of three-dimensional display (3D display). If an abnormal portion or the like is detected, the region where the right and left tomograms that have been displayed so far is switched to a window showing the three-dimensional display including the abnormal portion instead of the tomograms as shown in Fig. 6. This window arranges and displays a sagittal image on the upper left side on the upper stage in the screen, an axial image at the center on the upper stage in the screen to the right thereof, a coronal image on the lower left on the lower stage in the screen below the sagittal screen, and a tomogram in an arbitrary direction at the center on the lower stage in the screen to the right thereof. According to the ultrasound breast diagnostic system 100 as above, any abnormal portion, if detected, can be displayed in a three-dimensional manner or the like.

In this three-dimensional display means, means for indicating and determining a point of interest (point to be three-dimensionally scrutinized) on the screen is provided. When the point to be three-dimensionally scrutinized is indicated and determined using this means, as mentioned above, the "three-dimensional display" is made as in Fig. 6. On this screen, a scale is displayed movably. Therefore, the size of the lesion portion and a distance from the mammilla can be measured. Also, if the point of interest is identified as a positive candidate region by the lesion portion automatic detecting / marking device 40, since the region including the point is labeled, data relating to the size and the like can be immediately displayed.

The lesion portion automatic detecting / marking device 40 is a device that analyzes the voxel data of each of the right and left breasts with computer, automatically detects a lesion portion as a positive candidate and displays a mark indicating the positive candidate superimposed on the image, and the device is integrated with the display device used for interpretation of images 30 in this embodiment.

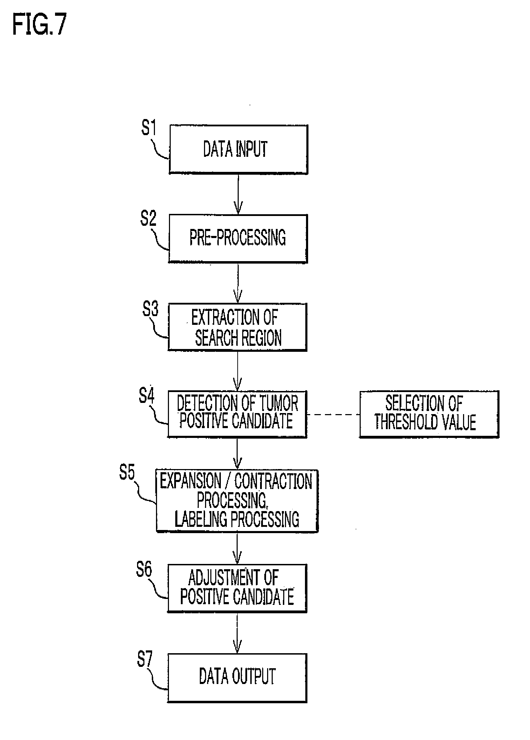

Fig. 7 is a flowchart for automatic detection of a lesion portion positive candidate. As shown in Fig. 7, the lesion portion automatic detection includes a data input step S1, a pre-processing step S2, an extraction step S3 of a search region (breast region), a detection step S4 of tumor positive candidate, an expansion / contraction processing and labeling processing step S5, an adjustment step S6 of positive candidate, and a data output step S7.

At Step S1, the voxel data of the breast is inputted. The inputted data is temporarily recorded in an appropriate buffer and stored during image diagnosis of the subject. In this voxel data, a position of the mammilla (mammilla center) has been acquired in advance.

Then, in the pre-processing step S2, a smoothing filter of 5 x 5 x 5 is applied to the voxel data for "noise removal". In an embodiment, saturation of a bright region and a dark region of an image is avoided using a fuzzy enhancement method. In addition, a speckle in the image can be removed using multiscale morphology.

At the search region extraction step S3, a region range (breast region) for detecting a tumor is extracted and determined. The breast surface (or a thin film in close contact with the surface) is represented as a continuous layer of a high echo. The pectoral muscle is visualized as a layer structure of a large quantity of (parallel) high echo and low echo substantially along the body. A method of detecting them includes three-dimensional differentiation filter, Sobel filter (weighted differentiation filter), Laplacian filter (secondary differentiation filter) (edge detection), local pattern matching, concentration gradient vector, binarization and the like. The breast region where a tumor is to be detected shall be a range in the rear of the breast surface and in front of the pectoral muscle.

At the tumor positive candidate detection step S4, the extracted breast region is searched for detecting a tumor. The first step in this detection is binarization processing. Since the tumor is represented as a shade with an echo lower than the periphery, a voxel (pixel) value of the tumor is set as a threshold value and the voxels below and exceeding the value are binarized.

Here, if the threshold value is too large, the number of false positive candidates is increased. On the contrary, if the value is too small, a true positive candidate might not be detected. Thus, the threshold value should be determined appropriately, but an image quality of a tomogram of a breast is different depending on the person or the age even if an imaging condition is the same. Thus, in this embodiment, a desired value can be selected as the threshold value on the basis of determination by a doctor. Thereby, a numerical balance between true positive and false positive can be set to the most suitable condition for the doctor. The plurality of threshold values offered for the selection can be a plurality of or continuous fixed threshold values examined and determined in advance or may be threshold values in some stages automatically created by obtaining a histogram of normal appropriate regions in the breast image and using information obtained from that in some way. Levels of the arbitrary threshold values from level 1 to level 5, for example, are selectably displayed on the screen.

Contraction processing in the expansion / contraction processing step S5 is processing of converting a black pixel having a white pixel in the vicinity to a white pixel for all the pixels (voxels). The expansion processing is processing of converting a white pixel having a black pixel in the vicinity to a black pixel by reversing the definition of the vicinity. Such processing is executed because the binarized image includes a macular point in a normal tissue other than a tumor candidate portion. The expansion and contraction processing is repeated appropriately, and a tumor which has become a mass is extracted. The labeling processing is applied to that so as to discriminate each tumor candidate.

The positive candidate adjustment step S6 finally determines a mass larger than a predetermined size (4 to 6 mm in general) such as 5 mm or more, for example, as a positive candidate. In this detection, the voxel number of each labeled candidate region is acquired and if it is not less than a predetermined value, it is determined as a positive candidate. Alternatively, a minimum diameter of each candidate region is acquired from a coordinate value and if it is not less than 5 mm, for example, it is determined as a positive candidate. Moreover, the detection can be also made by mapping using a 5mm-cube or -ball as a search box. The region with a low or no echo in the rear of the mammilla is extracted as a tumor candidate region all the time in this flow method, but that can be excluded at this stage. Finally, at the data output step S7, the extracted and determined positive candidate region (tumor) is stored with the coordinates and the data is outputted when the tomogram is displayed, later.

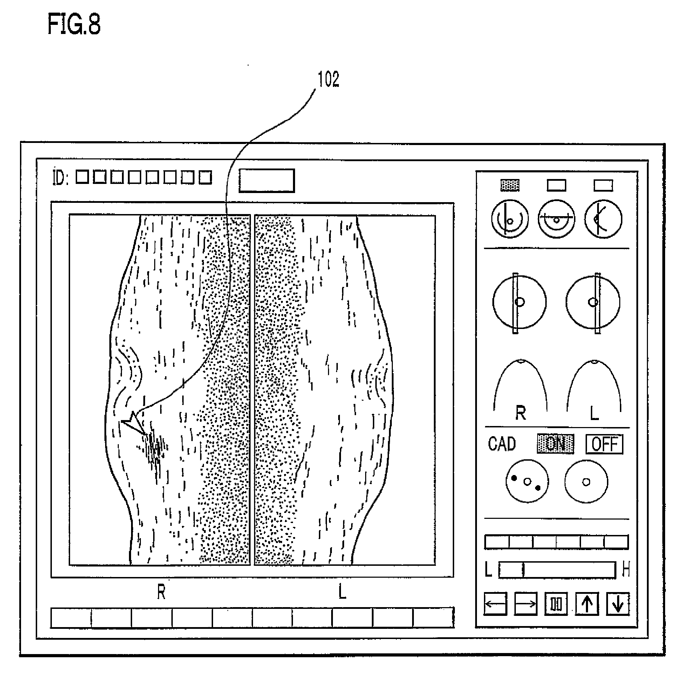

A positive candidate marking portion of the lesion portion automatic detecting / marking device 40 gives an indication mark indicating the positive candidate in superposition on the image on the basis of data relating to the position (coordinates) and range (size) of the positive candidate. Fig. 8 shows a tomogram formed as above, and an indication mark 102 is constituted in a form of an "arrow" as shown. This indication mark can be in any other arbitrary form such as an oval surrounding the positive candidate with an appropriate distance but in any form, its shape and arrangement should be given consideration so that the positive candidate can be surely indicated and the mark does not interfere with diagnosis itself of the positive candidate.

The CAD display of "ON" and "OFF" by the lesion portion automatic detecting / marking device 40 can be appropriately selected by an examiner such as a doctor and the like by clicking on CAD "ON" on the operation panel window. If the "ON" state of the CAD display is selected, first, the positive candidates of the tumor detected by the lesion portion automatic detecting / marking device 40 are all displayed by dots at applicable spots of a body mark of a breast (at least either of a half-moon-shaped side face mark and a circular plane mark). That is, in the illustrated embodiment, a "CAD" window portion is provided, in which a circular breast mark is displayed, and the positive candidates of the tumor are indicated by dots within this mark. As the breast mark displaying all the positive candidates in a list, use of a breast mark for position display of the cross section is possible.

A second possible way is to display the indication mark 102 indicating the detected positive candidate on the breast tomograms displayed symmetrically adjacently in superposition. This "positive candidate indication mark" is, as mentioned above, typically an arrow or a surrounding line. They are displayed sufficiently away from the positive candidate region or sufficiently proximate for indication of the region. The display of the indication mark 102 shall be selectable considering that the mark can be even confusing for some doctors.

That is, in the case of primary screening, the most serious burden for doctors is to prevent oversight of a lesion portion for the first place. In this regard, according to the lesion portion automatic detecting / marking device 40 in this embodiment, any suspicious shade as a lesion portion is indicated as a positive candidate as mentioned above, and the doctors can effectively use the information. Also, though lesion portion detection accuracy by the lesion portion automatic detecting / marking device 40 can not be perfect, the doctors can pay attention only to the shades that can not be detected by the lesion portion automatic detecting / marking device 40, considering the characteristics or features. This will extremely alleviate the burden on the doctors. As mentioned above, according to the ultrasound breast diagnostic system 100 of this embodiment, the burden on the doctor in image diagnosis can be minimized, by which capabilities of the doctor can be maximized.

The ultrasound breast diagnostic system 100 of this embodiment described above is particularly suitable for primary screening of breast cancer. That is, a quantity of the ultrasound tomograms collected by the ultrasound breast image pickup device 10 becomes enormous if the number of subjects is large, which is a serious burden on doctors who diagnose the images. Moreover, the image examination of sequentially displayed tomograms intensifies the burden. However, according to the ultrasound breast diagnostic system 100 of this embodiment, the tomograms of bilaterally symmetric cross-sectional spots of the right and left breasts to be diagnosed, which are sequentially displayed, are displayed symmetrically adjacently. Thus, since the presence of a lesion portion as an abnormal shade can be diagnosed on the basis of a difference or change between the right-side image and the left-side image in the tomograms, accurate diagnosis can be made more easily. That is, even for the ultrasound image in which image examination itself is not easy and the number of images to be examined is large, a burden on a doctor in image examination can be minimized.

The best mode of the ultrasound breast diagnostic system 100 has been described above, but the present invention is not limited to the configuration described in the above embodiment but the configuration can be changed as appropriate in a range not departing from its gist.

For example, a comprehensive database for recording and storing the obtained ultrasound tomographic data may be constructed and a network using LAN and the like may be formed with an image examination division. Thereby, doctors can perform image diagnosis with the past ultrasound tomographic data of the subject or examination results by means of mammography.

### Brief Description of Drawings

[Fig. 1] Fig. 1 is an explanatory diagram illustrating an outline system configuration of an entire ultrasound breast diagnostic system of an embodiment of the present invention.
[Fig. 2] Fig. 2 is an explanatory diagram illustrating an outline configuration of an ultrasound breast image pickup device of the embodiment.
[Fig. 3] Fig. 3 is an explanatory diagram illustrating a mechanical scanning path of an ultrasound probe of the embodiment.
[Fig. 4] Fig. 4 is a diagram illustrating a hardware configuration diagram of an display device used for interpretation of images and its display screen of the embodiment.
[Fig. 5] Fig. 5 is a diagram illustrating an example of another display screen of the display device used for interpretation of images.
[Fig. 6] Fig. 6 is a diagram illustrating an example of 3D display.
[Fig. 7] Fig. 7 is a flowchart for automatic detection of a lesion portion positive candidate.
[Fig. 8] Fig. 8 is a diagram illustrating the display screen in detection of a lesion portion by a lesion portion automatic detecting / marking device.

### Reference Numerals

- 10: ultrasound breast image pickup device
- 11: water bath
- 12: ultrasound probe
- 17: monitor
- 18: recording device
- 20: breast voxel data creating device
- 30: display device used for interpretation of images
- 40: lesion portion automatic detecting / marking device
- 100: ultrasound breast diagnostic system

## Claims

1. An ultrasound breast diagnostic system comprising:
an ultrasound breast image pickup device provided with a water bath in which a breast can be immersed downward and an ultrasound probe arranged at a bottom portion of the water bath, capable of performing mechanical scanning, for imaging of an entire region of each of the right and left breasts in a three-dimensional manner through transmission / reception of ultrasound waves;
a breast voxel data creating device for creating voxel data of each of the entire right and left breasts on the basis of image data obtained by the ultrasound breast image pickup device; and
an display device used for interpretation of images for displaying an image for image diagnosis on the basis of voxel data of each of the right and left breasts created by the breast voxel data creating device in which tomograms at bilaterally symmetric cross-sectional spots of the right and left breasts are sequentially displayed symmetrically adjacently and with a predetermined pitch from one end side to the other end side of the breast.

2. The ultrasound breast diagnostic system according to claim 1, wherein the tomograms of the right and left breasts displayed in the display device used for interpretation of images made up of either of a sagittal image and an axial image and they are displayed symmetrically adjacently with base portion sides of the breasts abutting each other.

3. The ultrasound breast diagnostic system according to claim 1, wherein the tomograms of the right and left breasts displayed in the display device used for interpretation of images are made up of coronal images.

4. The ultrasound breast diagnostic system according to any one of claims 1 to 3, wherein the tomograms of the right and left breasts displayed in the display device used for interpretation of images are made up of tomograms at bilaterally symmetric cross-sectional spots using a mammilla as a reference point.

5. The ultrasound breast diagnostic system according to any one of claims 1 to 4, further comprising a lesion portion automatic detecting / marking device that analyzes the breast voxel data of each of the right and left breasts with computer and automatically detects a lesion portion as a positive candidate and displays a mark indicating the positive candidate overlaid on the image.
